(19) Europäisches Patentamt / European Patent Office / Office européen des brevets

(11) **EP 1 785 129 A2**

(12) **DEMANDE DE BREVET EUROPEEN**

(43) Date de publication:
**16.05.2007 Bulletin 2007/20**

(51) Int Cl.:
*A61K 8/29* (2006.01)    *A61K 8/04* (2006.01)
*A61Q 19/02* (2006.01)

(21) Numéro de dépôt: **06291441.1**

(22) Date de dépôt: **13.09.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **14.09.2005 FR 0509370**

(71) Demandeur: **L'Oreal**
**75008 Paris (FR)**

(72) Inventeur: **Cassin, Guillaume**
**91140 Villebon Sur Yvette (FR)**

(74) Mandataire: **Dossmann, Gérard**
**Bureau Casalonga & Josse**
**Bayerstrasse 71/73**
**80335 München (DE)**

(54) **Composition cosmétique comprenant un actif et des particules contenant au moins un pigment inorganique coloré dans une matrice, et utilisation en soin de la peau.**

(57)    La présente demande concerne une composition cosmétique de traitement ou de soin de la peau qui comprend, dans un milieu physiologiquement acceptable, au moins un actif cosmétique et des particules colorées constituées d'au moins un pigment inorganique disposé à l'intérieur d'une matrice, ladite composition présentant une opacité inférieure ou égale à 35%. La présente demande concerne encore un procédé de traitement cosmétique mettant en oeuvre cette composition ainsi que les utilisations de cette composition notamment pour éclaircir ou homogénéiser le teint de la peau et pour corriger l'apparence des taches et des dyschromies de la peau.

EP 1 785 129 A2

**Description**

La présente invention se rapporte à de nouvelles compositions cosmétiques de traitement ou de soin de la peau, en particulier à des compositions destinées à conférer à la peau une couleur homogène. Les compositions selon la présente demande sont non couvrantes et comprennent au moins un principe actif et des particules colorées constituées d'au moins un pigment inorganique disposé à l'intérieur d'une matrice.

Il est fréquent que les personnes ayant une peau colorée, ou présentant des taches pigmentaires, de la couperose, ou des cernes, désirent corriger ces dyschromies cutanées et utilisent dans ce but des compositions cosmétiques ou dermatologiques permettant d'éclaircir et homogénéiser le teint. A cette fin, il est connu d'utiliser des compositions cosmétiques contenant des agents de blanchiment. Toutefois, il est nécessaire d'utiliser ces agents de façon prolongée et en des quantités élevées pour observer un effet de blanchiment de la peau car on n'observe pas un effet immédiat à l'application de ces compositions.

Il est connu d'utiliser dans un but d'éclaircissement et d'homogénéisation immédiate du teint des compositions cosmétiques contenant des composés fluorescents, on pense en particulier aux azurants optiques. Hélas, ces composés ne procurent un effet d'éclaircissement immédiat que dans des conditions d'éclairage optimum (lumière naturelle de forte intensité).

Il est aussi connu d'utiliser dans un but d'éclaircissement et d'homogénéisation immédiat du teint des produits couvrants qui, bien que dissimulant les imperfections cutanées, présentent l'inconvénient majeur de masquer l'aspect naturel de la peau (sensation de masque).

Il est enfin connu d'utiliser des produits contenant des pigments interférentiels qui bien que pouvant dissimuler les imperfections cutanées, présentent l'inconvénient majeur de donner un aspect brillant et peu naturel à la peau.

Il demeure donc un besoin de disposer de compositions cosmétiques non couvrantes présentant un effet d'éclaircissement ou d'homogénéisation du teint quelque soit la nature de l'éclairement, et qui ne donnent pas un aspect brillant et /ou peu naturel à la peau.

Le but de la présente invention est de proposer une composition cosmétique destinée au traitement et/ou au soin de la peau qui ne présente pas les inconvénients des compositions de l'art antérieur, en particulier qui soit non couvrante et qui permette de corriger les dyschromies c'est-à-dire d'éclaircir ou homogénéiser le teint de la peau.

De manière avantageuse et inattendue, la demanderesse vient maintenant de découvrir que ce but peut être atteint au moyen de compositions comprenant au moins un actif cosmétique et des particules colorées constituées d'au moins un pigment inorganique disposé à l'intérieur d'une matrice, dans la suite du texte, ces particules sont désignées par le terme « premières particules colorées ».

Les particules colorées utilisées dans les compositions selon la présente demande ont déjà été utilisées dans l'art antérieur par exemple dans la demande US 2005-0129638, dans des compositions pour le maquillage de la peau. Cette demande de l'art antérieur ne divulgue cependant pas des compositions de soin de la peau telles que les compositions conformes à l'invention qui, bien qu'étant non couvrantes permettent de conférer un teint de peau uniforme.

Les compositions selon l'invention permettent de corriger les dyschromies et notamment d'obtenir un effet anti-cerne immédiat ou encore un effet d'éclaircissement immédiat du teint.

Les compositions selon la présente invention présentent également l'avantage d'être non couvrantes et de ne pas donner un aspect brillant et peu naturel à la peau.

Selon une première variante de l'invention ladite composition cosmétique destinée au traitement et/ou au soin de la peau comprend, dans un milieu physiologiquement acceptable :

(i) au moins une première particule colorée constituée d'au moins un pigment inorganique disposé à l'intérieur d'une matrice,
(ii) au moins un actif cosmétique blanchissant de la peau, ladite composition présentant une opacité inférieure ou égale à 35%.

Les compositions conformes à cette première variante de l'invention permettent l'obtention d'une coloration homogène du teint non seulement immédiatement après application mais également de plus longue durée.

Selon une deuxième variante de l'invention ladite composition cosmétique destinée au traitement et/ou au soin de la peau comprend, dans un milieu physiologiquement acceptable,

(i) une phase continue aqueuse,
(ii) au moins un actif cosmétique de la peau, et
(iii) au moins une première particule colorée constituée d'au moins un pigment inorganique disposé à l'intérieur d'une matrice,
ladite composition présentant une opacité inférieure ou égale à 35%.

Il est souvent difficile d'obtenir une bonne dispersion des pigments inorganiques en phase aqueuse, en conséquence il est difficile d'obtenir des compositions homogènes, en particulier lorsque la concentration en pigments de ces compositions est faible. Les compositions conformes à cette deuxième variante de l'invention comprennent une phase continue aqueuse et des pigments inorganiques encapsulés dans une matrice, ces compositions présentent l'avantage d'être homogènes et de permettre l'obtention d'une coloration homogène du teint.

Selon une troisième variante de l'invention ladite composition cosmétique destinée au traitement et/ou au soin de la peau comprend, dans un milieu physiologiquement acceptable,

(i) au moins un actif cosmétique de la peau, et
(ii) au moins une première particule colorée constituée d'au moins deux pigments inorganiques de couleurs différentes disposés à l'intérieur d'une matrice,
ladite composition présentant une opacité inférieure ou égale à 35%.

Généralement lorsque l'on souhaite obtenir une composition comprenant un mélange de pigments inorganiques, il est nécessaire de préparer une pâte pigmentaire que l'on introduit dans une composition de base, or l'obtention d'une pâte pigmentaire homogène est souvent difficile à réaliser notamment lorsque l'on souhaite obtenir une composition de faible concentration en pigments. L'utilisation de particules colorées constituées d'au moins deux pigments inorganiques disposés à l'intérieur d'une matrice permet de s'affranchir de l'étape de réalisation d'un pâte pigmentaire, les compositions conformes à cette troisième variante de l'invention présentent l'avantage d'être homogènes et de permettre l'obtention d'une coloration homogène du teint.

De manière préférée, l'actif cosmétique de la peau utilisé dans les compositions conformes aux deuxième et troisième variantes de l'invention est un actif cosmétique blanchissant de la peau, ces compositions permettent l'obtention d'une coloration homogène du teint non seulement immédiatement après application mais également de plus longue durée.

De manière préférée, les compositions conformes aux première et troisième variantes de l'invention présentent une phase continue aqueuse.

D'autres caractéristiques, aspects, objets et avantages de la présente invention apparaîtront encore plus clairement à la lecture de la description et des exemples qui suivent.

Les compositions selon la présente demande sont des compositions de traitement ou le soin de la peau destinées à être utilisées pour éclaircir ou homogénéiser le teint. Elles sont non couvrantes, c'est-à-dire qu'elles laissent apparaître le grain de la peau tout en en masquant les imperfections, plus généralement elles permettent de corriger les dyschromies de la peau.

Les compositions selon la présente demande présentent une opacité inférieure à 35%, allant généralement de 5% à 35%, de préférence allant de 10 % à 30%.

Les compositions selon la présente demande présentent un écart de couleur (ΔE) après application sur carte de contraste (Erichsen 24/5) supérieur à 30.

En outre la teneur en pigments inorganiques libres des compositions conformes à l'invention est très faible, avantageusement cette teneur est inférieure à 0,2% en poids par rapport au poids total de la composition cosmétique.

Les compositions selon la présente demande bien qu'étant non couvrantes permettent de conférer un teint de peau homogène et de corriger les dyschromies de la peau de manière satisfaisante.

Les compositions selon la présente demande comprennent des premières particules comprenant au moins un pigment inorganique coloré disposé à l'intérieur d'une matrice.

Ainsi, la définition des « premières particules» utilisées selon l'invention n'englobe pas les particules qui sont couvertes par une couche ou plusieurs couches de pigments sur leur surface. Les « premières particules colorées » se distinguent notamment des particules, notamment lamellaires, à structure multicouche interférentielle.

Par « premières particules colorées » au sens de la présente demande, on entend des particules présentant une couleur autre que le blanc.

Dans les premières particules selon l'invention, les pigments inorganiques sont dispersés de façon homogène à l'intérieur de la matrice constituant lesdites particules, notamment sous forme de particules isolées.

Les pigments inorganiques sont notamment des matières colorantes insolubles dans la matrice dans laquelle ils sont disposés.

De préférence, les compositions conformes à l'invention contiennent des premières particules colorées constituées d'au moins deux, de manière préférée de deux ou trois pigments inorganiques de couleurs différentes disposés à l'intérieur d'une matrice.

Les compositions conformes à l'invention ont l'avantage de posséder une couleur homogène, définie avec précision et aisément reproductible même lorsque la concentration en particules composites est faible.

Ces compositions garantissent à l'utilisateur l'obtention d'une coloration précise et uniforme du support sur lesquelles elles sont appliquées, le support est ainsi coloré uniformément dans la teinte précise que l'utilisateur a choisi au préalable.

Les procédés permettant d'obtenir ce type de particules sont, par exemple, décrits dans les documents : JP-B-2591946 (Sumitomo Chemicals), JP-B2861806 (NSG), JP-A-H08-239310 (NSG), JP-A-H06-47273 (Suzuki Oil and Fat) dans le cas où le matériau formant les particules est inorganique, et entre autre dans le document JP-A-2001-354776 (Shin Etsu Chemicals) dans le cas où le matériau formant les particules est organique.

Par "pigments inorganiques colorés" on entend des pigments d'oxyde métallique dont la couleur peut être rouge, jaune, bleue, verte, violette, brune ou noire. Les exemples des pigments inorganiques colorés incluent l'oxyde de fer, l'ultramarine, le bleu de Prusse, le bleu ferrique, le violet de manganèse et l'oxyde de chrome. D'autres exemples de pigments inorganiques colorés incluent des pigments composites ou mixtes comprenant un mélange d'oxydes métalliques comme des oxydes mixtes de fer et de titane. Ces pigments ont, une taille allant de 0,1 à 1,5 microns.

Les particules composites utilisées selon l'invention peuvent en outre contenir un mélange de pigments inorganiques colorés ou un mélange de pigments inorganiques colorés et de pigments inorganiques blancs. Avantageusement, les particules composites utilisées selon l'invention contiennent un mélange de trois pigments inorganiques : un pigment inorganique rouge, un pigment inorganique jaune et un pigment inorganique blanc. Avantageusement, les pigments inorganiques à l'intérieur de la matrice des premières particules colorées sont présents en une teneur allant de 3 % à 60 % en poids par rapport au poids total des premières particules colorées.

Il n'y a aucune limitation en termes de forme des premières particules utilisées selon l'invention. Elles peuvent être, par exemple, de forme sphérique, avoir la forme d'un flocon, d'un cylindre plein ou d'une aiguille. Les premières particules utilisées selon l'invention peuvent être poreuses ou non poreuses. De manière préférée, au moins un des constituants des premières particules utilisées selon l'invention doit posséder un indice de réfraction inférieur à 1,8 voire inférieur à 1,6. Les premières particules utilisées selon l'invention ont une taille moyenne en volume allant de 1 à 40 microns mesurée par un granulomètre laser, tel que par exemple le Mastersizer 2000® de Malvernet le BI90+ ® de Broockhaven Instrument Corporation.

La matrice des premières particules peut être composée de tout matériau adéquat tel que par exemple du polymethylmethacrylate, de la cellulose, de la silice poreuse.

On peut citer comme exemples de premières particules contenant au moins un pigment inorganique coloré disponibles commercialement, les particules de polymethylmethacrylate notamment vendues sous la dénomination Ganzpearl par la société Ganz Chemicals, les particules de cellulose notamment vendues sous la dénomination Cellulobeads par la société Daito, les particules de silice poreuse notamment vendues sous la dénomination PC Ball par la société Miyoshi et les particules de silice poreuse notamment vendues sous la dénomination God Ball PC-LS par la société Suzuki Oil and Fat.

La composition selon l'invention comprend de 0,1 % à 10 %, et de préférence de 0,5% à 5 % en poids de premières particules colorées par rapport au poids total de la composition.

L'actif cosmétique (blanchissant) selon la présente demande est un actif cosmétique (blanchissant) de la peau.

Parmi les principes actifs cosmétiques utilisables dans les compositions selon la présente demande, on compte les antioxydants, les filtres solaires, les vitamines, les actifs hydratants, les composés auto-bronzants, les actifs antirides, les actifs hydrophiles ou lipophiles, les agents anti-pollution ou anti-radicaux libres, les actifs dermo-relaxants, les agents apaisants, les agents stimulant la synthèse de macromolécules dermiques ou épidermiques et/ou empêchant leur dégradation, les agents anti-glycation, les agents anti-irritants, les agents desquamants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

De manière particulière, les compositions selon la présente demande contiennent un actif cosmétique anti-âge choisi parmi les composés augmentant la synthèse de collagène, les composés inhibant la dégradation du collagène, les agents dermo-décontractants, les agents inhibant la glycation des protéines, les agents augmentant la prolifération des kératinocytes, les agents augmentant la différenciation des kératinocytes, et leurs mélanges.

Parmi les principes actifs cosmétiques blanchissant utilisables dans les compositions selon la présente demande, on compte les agents dépigmentant, anti-pigmentant ou pro-pigmentant et leurs mélanges.

Les agents dépigmentants ou anti-pigmentants susceptibles d'être incorporés dans la composition selon la présente demande comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.

Comme agent pro-pigmentant, on peut citer l'extrait de pimprenelle (Sanguisorba officinalis) commercialisé par la société MARUZEN et les extraits de chrysanthème (Chrysanthemum morifolium).

Parmi les principes actifs cosmétiques anti-âge utilisables dans les compositions selon la présente demande, on compte les composés suivants :

- les composés augmentant la synthèse de collagène (tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes) et/ou d'élastine (tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie®) et/ou des glycosaminoglycannes (tels que le produit de fermentation du lait par Lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin®) et/ou des protéoglycannes et/ou de la fibronectine (tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®) et/ou de la laminine ;
- les composés inhibant la dégradation du collagène (tels que les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja commercialisés par exemple par la société ICHIMARU PHARCOS sous la dénomination commerciale Flavostérone SB®, de trèfle rouge, de lin, de kakkon ou de sauge) et/ou de l'élastine (tels que l'extrait peptidique de graines de Pisum sativum commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique) ;
- les agents dermo-décontractants, tels que l'alvérine et ses sels, les sels de manganèse et en particulier le gluconate de manganèse, les sels de magnésium et en particulier le gluconate et le sulfate de magnésium, l'hexapeptide Argireline R commercialisé par la société LIPOTEC, l'adénosine, ainsi que les sapogénines et les extraits naturels, en particulier de Dioscorea opposita ou de Dioscorea villosa (Wild Yam) en contenant, ainsi que les extraits de Boswellia serrata ;
- les agents inhibant la glycation des protéines, tels que les extraits végétaux de la famille des Ericaceae, notamment un extrait de myrtille (Vaccinium angusfifollium) ; l'ergothionéine et ses dérivés ; et les hydroxystilbènes et leurs dérivés, tels que le resvératrol et le 3,3', 5,5'-tétrahydroxystilbène ;
- les agents augmentant la prolifération des kératinocytes (tels que les rétinoïdes dont le rétinol et le palmitate de rétinyle, l'adénosine, le phloroglucinol, les extraits de tourteaux de noix commercialisés par la société GATTEFOSSE et les extraits de Solanum tuberosum commercialisés par la société SEDERMA) et/ou des fibroblastes (tels que les protéines ou polypeptides végétaux, extraits notamment du soja, et les hormones végétales telles que les giberrellines et les cytokinines) ;
- les agents augmentant la différenciation des kératinocytes, tels que les minéraux dont le calcium ; un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de Voandzeia substerranea tel que celui commercialisé par la société Laboratoires Sérobiologiques sous la dénomination commerciale Filladyn LS 9397® ; et les lignanes tels que le sécoisolaricirésinol ;
et leurs mélanges.

Parmi ces composés, on préfère tout particulièrement l'acide ascorbique et/ou ses dérivés tels que le glucoside d'ascorbyle, l'adénosine et leurs mélanges.

La composition selon l'invention comprend de 0,001 à 10 %, de préférence de 0,01 à 5 % en poids d'au moins de principe actif blanchissant par rapport au poids total de la composition.

Les compositions selon la présente demande peuvent également comprendre des secondes particules colorées additionnelles en une teneur inférieure ou égale à 0,2% en poids par rapport au poids total de la composition cosmétique.

Avantageusement les secondes particules colorées additionnelles sont choisies parmi les pigments organiques, les pigments inorganiques et leurs mélanges.

Les pigments inorganiques utilisés en tant que secondes particules colorées additionnelles sont des pigments inorganiques « libres », c'est-à-dire qu'ils ne sont pas incorporés dans une particule.

Les pigments inorganiques utilisables dans les compositions selon la présente demande sont des pigments d'oxyde métallique de couleur rouge, jaune, bleue, verte, violette, brune ou noire. Les pigments inorganiques colorés sont généralement choisis parmi l'oxyde de fer, l'ultramarine, le bleu de Prusse, le bleu ferrique, le violet de manganèse et l'oxyde de chrome, les oxydes mixtes de fer et de titane.

De manière préférée, les compositions selon la présente demande comprennent également des charges matifiantes et /ou des nacres de petite taille.

Au sens de l'invention, « charge matifiante » désigne une particule sphérique ou non, poreuse ou non, présentant un indice de réfraction inférieur ou égal à 2,2, notamment inférieur ou égal à 2, et en particulier inférieur ou égal à 1,8, de préférence allant de 1,3 à 1,6. Les « charges matifiantes » selon l'invention ont une taille en volume comparable aux nacres utilisées. La taille préférée des charges est donc inférieure à 15 microns mesurée par un granulomètre laser, tel que par exemple le Mastersizer 2000® de Malvern et le BI90 + de Broockhaven Instrument Corporation.

Dans un mode préférentiel de l'invention les « charges matifiantes » sont sphériques.

Dans un autre mode préférentiel de l'invention les « charges matifiantes » sont poreuses. Dans ce cas, la surface spécifique des particules -que l'on peut relier à la porosité- est supérieure à 10 m$^2$/g, de préférence supérieure à 50 m$^2$/g.

Le caractère matifiant des charges selon l'invention est défini par une mesure au gonioréflectomètre. Pour ceci, on étale la composition contenant 5% de charges sur une carte de contraste (Prufkarte type 24/5 - 250 cm$^2$ commercialisée par la société Erichsen) à l'aide d'un tire-film mécanique (épaisseur humide 30 microns). On sèche ensuite la composition pendant une nuit à une température de 37 °C, puis on mesure la réflexion à l'aide d'un gonioréflectomètre. Le résultat obtenu est le rapport R entre la réflexion spéculaire et la réflexion diffuse. La valeur de R est d'autant plus faible que l'effet matifiant est important. Les charges matifiantes selon l'invention sont celles qui à un taux de 5% dans une composition cosmétique donnent une valeur de R inférieure à 1, de préférence inférieure à 0,75.

Plus particulièrement, ces charges peuvent par exemple être choisie parmi :

- les microparticules poreuses de silice, comme par exemple les Silica beads SB150 et SB700 de Miyoshi de taille moyenne 5 microns; les Sunspheres Série-H d'Asahi Glass comme par exemple les Sunsphères H33, H51, et H53 de taille respectives 3, 5 et 5 microns ;
- les poudres de polytétrafluoroéthylène, comme les PTFE Ceridust 9205F de Clariant de taille moyenne 8 microns ;
- les poudres de résine de silicone comme les Silicon resin Tospearl 145A de GE Silicone de taille moyenne 4,5 microns ;
- les particules hémisphériques creuses de silicone comme celles décrites dans la demande de brevet EP 1 530 961 ;
- les poudres de copolymères acryliques, notamment de poly(méth)acrylate de méthyle, comme les particules PMMA Jurymer MBI de Nihon Junyoki de taille moyenne 8 microns, les sphères creuses de PMMA vendues sous la dénomination Covabead LH85 par la société Wackherr, et que les microsphères de chlorure de vinylidene/ acrylonitrile / methacrylate de methylene expansées vendues sous la dénomination Expancel ;
- les poudres de cire comme les particules Paraffin wax microease 114S de micropowders de taille moyenne 7 microns ;
- les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns) ;
- les poudres d'organopolysiloxane élastomérique réticulé enrobées de résine de silicone, notamment de résine silsesquioxane, comme décrit par exemple dans le brevet US 5 538 793. De telles poudres d'élastomère sont vendues sous les dénominations « KSP-100 », « KSP-101 », « KSP-102 », « KSP-103 », « KSP-104 », « KSP-105 » par la société SHIN ETSU ;
- les poudres composites de talc/dioxyde de titane/alumine/silice comme celles vendues sous la dénomination Coverleaf AR-80 par la société Catalyst & chemicals ;
- les poudres de polyamide (Nylon®), comme par exemple les particules de Nylon 12 du type Orgasol d'Atofina de taille moyenne 10 microns;
- et leurs mélanges.

Les taux introduits dépendent de l'effet recherché et vont de 0,1% à 15%, de préférence de 0,5% à 10%.

La composition selon l'invention peut en outre renfermer des nacres de petites tailles.

Au sens de l'invention, le terme « nacres » ou désigne des particules qui se présentent sous la forme d'une multitude de fines plaquettes à haut indice de réfraction qui chacune réfléchissent et transmettent partiellement la lumière incidente, ces particules sont également parfois nommées « pigments interférentiels ».

Les effets en terme de couleur obtenus sont liés à la structure lamellaire de ces particules et dérivent des lois physiques de l'optique des couches minces (voir par exemple : *Pearl Lustre Pigments - Physical principles, pro-*

*perties, applications - R. Maisch, M. Weigand. Verlag Moderne Industrie).* Les nacres employées selon l'invention sont des pigments de type multicouches basés sur un substrat naturel à base, entre autre, de mica recouvert d'une ou plusieurs couches d'oxydes métalliques.

Les nacres selon l'invention sont caractérisées par une taille moyenne en volume inférieure à 15 microns mesurée par un granulomètre laser, tel que par exemple le Mastersizer 2000® de Malvernet le BI90 +® de Broockhaven Instrument Corporation. On pense an particulier aux nacres mica/oxyde d'étain/oxyde de titane : Timiron Silk Blue®, Timiron Silk Red®, Timiron Silk Green®, Timiron Silk Gold® et Timiron Super Silk ® proposées par la société Merck et aux nacres mica/oxyde de fer/oxyde de titane : Flamenco Satin Blue®, Flamenco Satin Red®, et Flamenco Satin Violet® proposées par la société Engelhard.

La composition selon l'invention aussi renfermer diverses charges additionnelles d'origine minérale ou organique. Elles peuvent être de toute forme, notamment plaquettaires, sphériques ou oblongues, ou encore présenter une autre forme cristallographique (par exemple en feuillet, cubique, hexagonale, orthorhombique, etc...). Parmi les charges additionnelles utilisables dans la composition selon l'invention, on peut notamment citer l'oxychlorure de bismuth, le talc, le mica, le dioxyde de titane, le kaolin, les particules de poly-β-alanine et de polyéthylène, la lauroyl-lysine, l'amidon, le nitrure de bore, le carbonate de calcium précipité, le carbonate et l'hydrocarbonate de magnésium, le sulfate de baryum, l'hydroxyapatite, les microcapsules de verre ou de céramique et les savons métalliques dérivés d'acides organiques carboxyliques ayant de 8 à 22 atomes de carbone, notamment de 12 à 18 atomes de carbone, par exemple le stéarate de zinc, de magnésium ou de lithium, le laurate de zinc, le myristate de magnésium.

De préférence la composition selon l'invention renferme de l'oxychlorure de bismuth. On pense aux particules d'oxychlorure de bismuth commercialisées sous les dénominations BIRON LF 2000 et PEARL GLO SF par les sociétés MERCK et ENGELHARD, respectivement.

Les taux de nacres et de charges introduit dépendent de l'effet recherché et vont de 0,1% à 15%, de préférence de 0,1% à 15%, de manière encore préférée de 0,5% à 5%.

La composition conforme à l'invention peut également contenir au moins un agent choisi parmi les colorants, les pigments, les parfums, les conservateurs, les filtres solaires physiques et chimiques, les séquestrants, les actifs liposolubles ou hydrosolubles, les hydratants tels que les polyols et notamment la glycérine, les ajusteurs de pH (acides ou bases).

Les agents ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Les compositions cosmétiques décrites dans la présente invention sont des sérums, des gels aqueux, des émulsions, des sticks, des sprays. Par émulsion on entend des compositions comprenant au moins une phase aqueuse et au moins une phase huileuse dispersée l'une dans l'autre ainsi que les émulsions multiples eau dans huile dans eau ou bien huile dans eau dans huile.

La présente demande vise encore un procédé de traitement cosmétique -c'est-à-dire non-thérapeutique- des matières kératiniques, notamment de la peau, consistant à appliquer sur lesdites matières kératiniques une composition selon l'invention, et aussi l'utilisation d'une composition selon l'invention notamment pour réduire l'apparence des taches ou des dyschromies de la peau ou pour éclaircir ou homogénéiser le teint de la peau.

Les exemples qui suivent servent à illustrer l'invention sans toutefois présenter un caractère limitatif.

EXEMPLES

I. Préparation des compositions

On prépare les compositions des exemples selon le mode opératoire suivant :

-     chauffer la phase B à environ 75°C et y incorporer le Polyacryldimethyltauramide d'ammonium; agiter jusqu'à obtention d'un gel homogène.
-     chauffer la phase A à environ 75°C.
-     réaliser l'émulsion en incorporant la phase A dans la phase B.
-     à 40-45°C, incorporer la phase C et maintenir l'agitation jusqu'à refroidissement complet.

II. Exemples de compositions cosmétiques de soin de la peau

**Exemple 1 : composition cosmétique :**

A-
Glyceryl stearate (et) PEG-100 stearate :          2.00 g
Dimyristyl tartrate (et) alcool cetearylique
(et) C12-15 pareth-7 (et) PPG-25 laureth-25:          1.50 g
Cyclohexasiloxane :          10.00 g
Alcool Stearylique :          1.00 g

B-
Eau :          80.25 g
Vitamine C :          0.50 g
Phenoxyethanol :          1.00 g
Pentasodium ethylene diamine
tetramethylene phosphate :          0.05 g
Polyacryldimethyltauramide d'ammonium:          0.40 g
Xanthan gum :          0.20 g

C-
God Ball PC-LS-19
(oxyde de fer et oxyde de titane encapsulés dans de la silice
($TiO_2$ 45.8 % - silice 50% - Oxyde de fer 4.2%)          3.00 g

Cette composition présente une valeur $\Delta E$ = 42,3 et une valeur d'opacité de 22,9%.

**Exemple 2 : composition cosmétique**

A-
Glyceryl stearate (et) PEG-100 stearate :          2.00 g
Dimyristyl tartrate (et) alcool cetearylique
(et) C12-15 pareth-7 (et) PPG-25 laureth-25:          1.50 g
Cyclohexasiloxane :          10.00 g
Alcool Stearylique :          1.00 g

B-
Eau : 80.25 g
Vitamine C :          0.50 g
Phenoxyethanol :          1.00 g
Pentasodium ethylene diamine
tetramethylene phosphate :          0.05 g
Polyacryldimethyltauramide d' ammonium:          0.40 g
Xanthan gum :          0.20 g

C-
God Ball PC-LS-14
(oxyde de fer et oxyde de titane
encapsulés dans de la silice
($TiO_2$ 44.7 % - silice 50% - Oxyde de fer 5.3%)          3.00 g

Cette composition présente une valeur $\Delta E$ = 42,5 et une valeur d'opacité de 22,8%.

**Exemple 3 : composition cosmétique**

A-
Glyceryl stearate (et) PEG-100 stearate : 2.00 g Dimyristyl tartrate (et) alcool cetearylique
(et) C12-15 pareth-7 (et) PPG-25 laureth-25: 1.50 g
Cyclohexasiloxane : 10.00 g
Alcool Stearylique : 1.00 g

B-
Eau : 70.25 g
Acide ellagique 0.50 g
Phenoxyethanol : 1.00 g
Pentasodium ethylene diamine tetramethylene phosphate : 0.05 g
Polyacryldimethyltauramide d'ammonium: 0.40 g
Xanthan gum : 0.20 g

C-
God Ball PC-LS-14
(oxyde de fer et oxyde de titane encapsulés dans
de la silice (TiO$_2$ 44.7 % - silice 50%
Oxyde de fer 5.3%) 2.00 g
Particules hemisphériques creuses de silicone
(NLK 506 - Takemoto Oil and Fats): 3.00 g

Cette composition présente une valeur ΔE = 35,3 et une valeur d'opacité de 29,8%.

**Exemple 4 : composition cosmétique**

A-
Glyceryl stearate (et) PEG-100 stearate : 2.00 g
Dimyristyl tartrate (et) alcool cetearylique
(et) C12-15 pareth-7 (et) PPG-25 laureth-25: 1.50 g
Cyclohexasiloxane : 10.00 g
Alcool Stearylique : 1.00 g

B-
Eau : 69.25g
Acide kojique 0.50 g
Phenoxyethanol : 1.00 g
Pentasodium ethylene diamine
tetramethylene phosphate : 0.05 g
Polyacryldimethyltauramide d'ammonium: 0.40 g
Xanthan gum : 0.20 g

C-
God Ball PC-LS-14
(oxyde de fer et oxyde de titane
encapsulés dans de la silice
(TiO$_2$ 44.7 % - silice 50% - Oxyde de fer 5.3%) 2.00 g
Particules hemisphériques creuses de silicone
(NLK 506 - Takemoto Oil and Fats): 2.00 g
Nacre mica / oxyde de titane / oxyde d'étain
(Timiron silk Blue): 1.00 g

Cette composition présente une valeur ΔE = 42 et une valeur d'opacité de 23,2%.

III. Mise en évidence de l'aspect non couvrant des compositions selon l'invention

Les compositions telles que décrites ci-dessus sont caractérisées par un écart de couleur (ΔE) supérieur à 30.

Pour déterminer cette valeur, on étale la composition sur un film transparent (Hp Color laser jet transparency, Hp Invent ; CP2936A) au moyen d'un applicateur automatique de chez Braive instruments (épaisseur humide 50 μm). Les étalements sont ensuite disposés à l'étuve thermostatée et ventilée 24 heures à 37°C.

Une fois séchés ces films sont déposés pour évaluation sur une carte de contraste (Prufkarte type 24/5 - 250 cm$^2$ commercialisée par la société Erichsen). L'écart de couleur ΔE entre la partie noire et la partie blanche de la carte de contraste (mesuré au travers du film étudiés) a été ensuite mesuré avec un colorimètre Minolta CR-400. L'écart de couleur est obtenu à l'aide de la formule d'écart de couleur de Hunter dans l'espace colorimétrique L, a, b : $\Delta E = [(\Delta L)^2 + (\Delta a)^2 + (\Delta b)^2]^{0,5}$. Les valeurs obtenues sont une moyenne de 5 mesures.

IV. Protocole de mesure de l'opacité des compositions selon l'invention

Les compositions telles que décrites ci-dessus sont caractérisées par une opacité inférieure à 35 %.

Pour déterminer cette valeur, on étale la composition sur un film transparent (Hp Color laser jet transparency, Hp Invent ; CP2936A) au moyen d'un applicateur automatique de chez Braive instruments (épaisseur humide 50 μm). Les étalements sont ensuite disposés à l'étuve thermostatée et ventilée 24 heures à 37°C.

Une fois séchés ces films sont déposés pour évaluation sur une carte de contraste (Prufkarte type 24/5 - 250 cm$^2$ commercialisée par la société Erichsen). L'opacité est ensuite mesurée à l'aide d'un colorimètre Minolta CR-400 à partir des valeurs Y des parties noires et blanches obtenues dans le système tristimulaires (X, Y, Z). La valeur d'opacité est obtenue à partir de l'équation suivante

$$(\text{Y zone noire} / \text{Y zone blanche})* 100 = \text{Opacité \%}$$

Si le film est totalement transparent l'opacité est égale à 0.

Evaluation sensorielle :

Les effets éclaircissant et homogénéisant ainsi que et les aspects cosmétiques des compositions selon l'invention (exemples 1 à 4) ont été évalués sur un panel de 6 sujets présentant des dyschromies (taches pigmentaires, dépigmentation, taches de rousseur, couperose, cernes). Pour ces personnes, on observe après application des compositions selon l'invention des effets éclaircissant, unifiant, lisseur sur le grain de la peau, et les points noirs sont camouflés. Le teint est moins brouillé, les contrastes de couleur tels que les taches brunes et les cernes sont amoindries.

**Revendications**

1. Composition cosmétique destinée au traitement et/ou au soin de la peau comprenant, dans un milieu physiologiquement acceptable :

    (i) au moins une première particule colorée constituée d'au moins un pigment inorganique disposé à l'intérieur d'une matrice,
    (ii) au moins un actif cosmétique blanchissant de la peau, ladite composition présentant une opacité inférieure ou égale à 35%.

2. Composition cosmétique destinée au traitement et/ou au soin de la peau comprenant, dans un milieu physiologiquement acceptable,

    (iv) une phase continue aqueuse,
    (v) au moins un actif cosmétique de la peau, et
    (vi) au moins une première particule colorée constituée d'au moins un pigment inorganique disposé à l'intérieur d'une matrice,
    ladite composition présentant une opacité inférieure ou égale à 35%.

3. Composition cosmétique destinée au traitement et/ou au soin de la peau comprenant, dans un milieu physiologiquement acceptable,

   (iii) au moins un actif cosmétique de la peau, et
   (iv) au moins une première particule colorée constituée d'au moins deux pigments inorganiques de couleurs différentes disposés à l'intérieur d'une matrice,

   ladite composition présentant une opacité inférieure ou égale à 35%

4. Composition cosmétique selon la revendication 2 ou 3 **caractérisée en ce que** l'actif cosmétique est un actif cosmétique blanchissant.

5. Composition cosmétique selon la revendication 1 ou 4 **caractérisée en ce que** l'actif cosmétique blanchissant est choisi parmi les agents dépigmentant, anti-pigmentant ou pro-pigmentant et leurs mélanges.

6. Composition cosmétique selon l'une des revendications 1, 4 ou 5 **caractérisée en ce que** l'actif cosmétique blanchissant est présent en une teneur allant de 0,001% à 10%, de préférence allant de 0,01% à 5% en poids par rapport au poids total de la composition.

7. Composition cosmétique selon la revendication 1 ou 3 **caractérisée en ce qu'**elle présente une phase continue aqueuse.

8. Composition cosmétique selon l'une quelconque des revendications précédentes **caractérisée en ce qu'**elle présente une opacité allant de 5% à 35%, de préférence allant de 10% à 30%.

9. Composition cosmétique selon la revendication 1 ou 2 **caractérisée en ce que** les premières particules colorées sont constituées d'au moins deux pigments inorganiques de couleurs différentes disposés à l'intérieur d'une matrice.

10. Composition cosmétique selon l'une quelconque des revendications précédentes **caractérisée en ce que** les premières particules colorées sont constituées d'au moins trois pigments inorganiques de couleurs différentes disposés à l'intérieur d'une matrice.

11. Composition cosmétique selon l'une quelconque des revendications précédentes **caractérisée en ce qu'**elle comprend des secondes particules colorées additionnelles en une teneur inférieure ou égale à 0,2% en poids par rapport au poids total de la composition cosmétique.

12. Composition cosmétique selon la revendication précédente **caractérisée en ce que** les secondes particules colorées additionnelles sont choisies parmi les pigments organiques, les pigments inorganiques et leurs mélanges.

13. Composition cosmétique selon l'une quelconque des revendications précédentes **caractérisée en ce que** le ou les pigments inorganiques sont choisis parmi les pigments d'oxyde métallique de couleur rouge, jaune, bleue, verte, violette, brune ou noire.

14. Composition cosmétique selon l'une quelconque des revendications précédentes **caractérisée en ce que** le ou les pigments inorganiques colorés sont choisis parmi l'oxyde de fer, l'ultramarine, le bleu de Prusse, le bleu ferrique, le violet de manganèse, l'oxyde de chrome, les oxydes mixtes de fer et de titane.

15. Composition cosmétique selon l'une quelconque des revendications précédentes **caractérisée en ce que** les pigments inorganiques à l'intérieur de la matrice des premières particules colorées sont présents en une teneur allant de 3 % à 60 % en poids par rapport au poids total des premières particules colorées.

16. Composition cosmétique selon la revendication précédente **caractérisée en ce que** les premières particules colorées sont présentes en une teneur allant de 0,1 % à 10 %, de préférence de 0,5% à 5 % par rapport au poids total de la composition cosmétique.

17. Composition cosmétique selon l'une quelconque des revendications précédentes **caractérisée en ce qu'**elle comprend un actif cosmétique choisi parmi les antioxydants, les filtres solaires, les vitamines, les actifs hydratants, les composés auto-bronzants, les actifs antirides, les actifs hydrophiles ou lipophiles, les agents anti-pollution ou anti-radicaux libres, les actifs dermo-relaxants, les agents apaisants, les agents stimulant la synthèse de macromolécules dermiques ou épidermiques et/ou empêchant leur dégradation, les agents anti-glycation, les agents anti-irritants, les agents desquamants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

**18.** Composition cosmétique selon l'une quelconque des revendications précédentes **caractérisée en ce qu'**elle comprend un actif cosmétique anti-âge choisi parmi les composés augmentant la synthèse de collagène, les composés inhibant la dégradation du collagène, les agents dermo-décontractants, les agents inhibant la glycation des protéines, les agents augmentant la prolifération des kératinocytes, les agents augmentant la différenciation des kératinocytes, et leurs mélanges.

**19.** Composition cosmétique selon l'une quelconque des revendications précédentes **caractérisée en ce que** le ou les actifs cosmétiques sont présents en une teneur allant de 0,001% à 10%, de préférence allant de 0,01% à 5% en poids par rapport au poids total de la composition.

**20.** Composition cosmétique selon l'une quelconque des revendications précédentes **caractérisée en ce qu'**elle comprend en outre des charges matifiantes et /ou des nacres de petite taille.

**21.** Composition cosmétique selon la revendication précédente **caractérisée en ce qu'**elle comprend de 0,1% à 15% de charges matifiantes et /ou de nacres de petite taille en poids par rapport au poids total de la composition.

**22.** Composition cosmétique selon l'une quelconque des revendications précédentes **caractérisée en ce qu'**elle comprend en outre au moins un additif cosmétique choisi parmi les colorants, les agents gélifiants et/ou épaississants hydrophiles ou lipophiles, les parfums, les conservateurs, les neutralisants, les émollients, les séquestrants, les agents filmogènes, les tensioactifs et leurs mélanges.

**23.** Composition cosmétique selon l'une quelconque des revendications précédentes **caractérisée en ce qu'**elle se présente sous une forme choisie parmi les sérums, les gels aqueux, les émulsions, les sticks, les sprays.

**24.** Composition cosmétique selon l'une quelconque des revendications précédentes **caractérisée en ce qu'**elle est sous forme d'une émulsion huile dans eau.

**25.** Procédé de traitement cosmétique des matières kératiniques, notamment de la peau, consistant à appliquer sur lesdites matières kératiniques une composition selon l'une quelconque des revendications précédentes.

**26.** Utilisation d'une composition selon l'une quelconque des revendications 1 à 23 pour réduire l'apparence des taches ou des dyschromies de la peau.

**27.** Utilisation d'une composition selon l'une quelconque des revendications 1 à 23 pour éclaircir ou homogénéiser le teint de la peau.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20050129638 A
- JP 2591946 B
- JP 861806 B
- JP H08239310 A
- JP H0647273 A
- JP 2001354776 A
- EP 895779 A
- EP 524109 A
- WO 9910318 A
- WO 9932077 A
- WO 9922707 A
- EP 1530961 A
- US 5538793 A